# DEMANDE DE BREVET EUROPEEN

(11) **EP 4 494 567 A1**
(43) Date de publication de la demande: **22.01.2025**
(21) Numéro de dépôt: 24184391.1
(22) Date de dépôt: 25.06.2024
(51) Int. Cl.: A61B 8/00, G10K 11/00

(54) **SUPPORT D'ACCESSOIRE ET SYSTÈME ACCESSOIRE POUR SONDE ULTRASONORE**

(30) Priorité: 21.07.2023 FR 2307846
(71) Demandeur: SUPERSONIC IMAGINE, 13290 Aix-en-Provence (FR)
(72) Inventeur: MAJOREL, Damien, 13090 Aix-en-Provence (FR); ALBRAND, Fabien, 05310 La Roche-de-Rame (FR)
(74) Mandataire: RVDB Nantes

(57) **Abrégé**

La présente divulgation concerne un support d'accessoire (10) pour sonde ultrasonore comprenant : une première interface de fixation (12) configurée pour s'appairer mécaniquement à une sonde ultrasonore (2) ; et une deuxième interface de fixation (14), de forme complémentaire à une troisième interface de fixation (24) dont est pourvu un accessoire de sonde (20), configurée pour s'appairer mécaniquement (AP2) de façon amovible avec ladite troisième interface de fixation (24) par rotation de l'accessoire de sonde (20) relativement au support d'accessoire (10). La divulgation concerne également un système accessoire (SY1) comprenant un tel support d'accessoire (10) et un tel accessoire de sonde (20).

## Description

### Technique antérieure

La présente divulgation concerne les supports d'accessoire pour sonde ultrasonore et vise en particulier les supports et systèmes destinés à fixer un accessoire à une sonde à ultrasons.

Les dispositifs ultrasonores trouvent aujourd'hui de nombreuses applications, dans les domaines notamment de l'acoustique, de l'étude des matériaux, de l'imagerie médicale et de la biomédecine.

A cet effet, un dispositif à ultrasons comprend généralement une sonde ultrasonore pilotée au moyen de signaux électriques, ces signaux étant par exemple transmis entre la sonde et une unité de commande. La sonde ultrasonore (appelée aussi dispositif transducteur d'ultrasons) comprend au moins un ou un ensemble d'éléments transducteurs d'ultrasons destinés à émettre et/ou recevoir des ondes ultrasonores vers et/ou depuis un milieu d'intérêt. Ainsi, des signaux électriques représentant des ondes ultrasonores peuvent être transmis à et/ou reçus depuis ces éléments transducteurs, causant l'émission et/ou la réception d'ondes ultrasonores dans et/ou depuis le milieu considéré.

Plus particulièrement, dans un procédé conventionnel d'imagerie par ultrasons, on peut utiliser par exemple une sonde à ultrasons équipée d'un ou plusieurs transducteurs pour convertir les signaux électriques en ondes ultrasonores. Les transducteurs peuvent émettre un ou successivement plusieurs faisceaux ultrasonores en direction d'un milieu, ce qui correspond à une opération d'émission. Ensuite, dans une opération de réception, un ensemble de signaux d'écho rétrodiffusés sont reçus du milieu par le même ensemble ou par un autre ensemble d'éléments transducteurs. En particulier, chacun des éléments transducteurs peut par exemple convertir un signal d'écho reçu en un signal électrique. Le signal peut ensuite être traité par le système à ultrasons ou par tout système associé directement connecté ou non.

Dans certains cas d'usage, il peut être utile d'installer un accessoire sur la sonde ultrasonore. A titre d'exemple, il est connu de fixer un capteur de position sur une sonde échographique pour permettre à un système d'imagerie de détecter la position de la sonde afin d'aider l'utilisateur à positionner la sonde par rapport au milieu d'intérêt. Il est ainsi possible de superposer une image (par exemple une image échographique ou une image IRM (imagerie par résonance magnétique) avec une représentation de la sonde ou de sa position afin de repérer la position de la sonde dans le milieu considéré. Grâce à ce type de capteur, on peut identifier aisément une position donnée à l'image, à des fins par exemple d'observation, d'intervention chirurgicale, de biopsie ou autre.

Le bon positionnement d'un tel capteur par rapport à la sonde ultrasonore est critique pour permettre une localisation précise de la sonde dans l'espace. Tout déplacement inopiné du capteur sur la sonde peut en particulier se traduire par la détection par le système d'un mouvement parasite de la sonde qui ne reflète pas le comportement réel de la sonde.

Pour éviter la détection de tels mouvements parasites, il convient donc de fixer de façon fiable et robuste le capteur à la sonde ultrasonore. Toutefois, l'installation d'un tel capteur ne doit pas se faire au détriment d'autres contraintes, notamment en termes d'ergonomie, de facilité de mise en place et de nettoyage et de coût.

Afin de pallier tout problème de déplacement parasite, il est possible d'intégrer directement un capteur de position dans une sonde ultrasonore. Cependant, l'utilisateur de la sonde peut ne pas avoir toujours besoin de ce capteur qui par ailleurs entraîne une augmentation du poids de la sonde et diminue potentiellement son ergonomie, voire son autonomie. La présence du capteur dans la sonde peut notamment nécessiter de modifier la forme extérieure de la sonde, et/ou d'augmenter le diamètre du câble reliant la sonde à l'unité de commande ce qui peut conduire à une rigidité accrue de l'ensemble. En outre, il peut être souhaitable de changer le capteur selon l'utilisation qui est faite de la sonde ultrasonore ou d'utiliser un même capteur sur différentes sondes.

Il est connu d'utiliser un support de capteur qui permet de fixer de façon amovible un capteur de position à une sonde ultrasonore. Cependant, un tel support ne permet pas d'offrir une fixation amovible, fiable et robuste du capteur sur la sonde, tout en garantissant une bonne ergonomie à l'usage et cela à un coût limité. En cas de clipsage du capteur sur le support par exemple, il y a un risque que le capteur bouge ou se désolidarise de façon inopinée de la sonde. Si au contraire la fixation par clipsage requiert une force excessive pour fixer ou détacher le capteur de la sonde, cela peut poser des problèmes de manipulation à l'utilisateur, notamment si la surface de préhension du capteur est limitée ou si l'utilisateur porte des gants. En général, l'utilisateur a besoin de pouvoir positionner ou retirer rapidement et efficacement le capteur afin de limiter les risques d'endommager le matériels, optimiser le temps d'utilisation de la machine et éviter les manipulations inutiles.

En outre, il est généralement difficile de nettoyer de manière rapide et fiable un tel support de capteur en raison de sa conception peu adaptée, ce qui peut poser problème en particulier dans les environnements où les exigences en termes d'hygiène sont élevées, tel que notamment dans le domaine médical.

### Exposé de la divulgation

L'un des objets de la présente divulgation est de résoudre au moins l'un des problèmes ou déficiences décrits précédemment.

En particulier, un objet de la présente divulgation est d'offrir un support d'accessoire permettant d'assurer une fixation amovible d'un accessoire à une sonde ultrasonore, de sorte que cette fixation soit fiable et robuste tout en garantissant une bonne ergonomie et facilité d'utilisation.

A cet effet, selon un premier aspect, la présente divulgation vise un support d'accessoire pour sonde ultrasonore comprenant :
- une première interface de fixation configurée pour s'appairer mécaniquement à une sonde ultrasonore ; et
- une deuxième interface de fixation, de forme complémentaire à une troisième interface de fixation dont est pourvu un accessoire de sonde, configurée pour s'appairer mécaniquement de façon amovible avec ladite troisième interface de fixation par rotation de l'accessoire de sonde relativement au support d'accessoire.

Le support d'accessoire selon la divulgation peut comporter d'autres caractéristiques qui peuvent être prises séparément ou en combinaison, notamment parmi les modes de réalisation qui suivent qui sont présentés à titre d'illustration uniquement et peuvent être combinés ou associés sauf stipulation contraire.

Selon un exemple, l'une parmi les deuxième et troisième interfaces de fixation constitue un élément mâle et l'autre constitue un élément femelle, lesdits éléments mâle et femelle étant configurés pour s'appairer mécaniquement en rotation par insertion de l'élément mâle dans l'élément femelle.

Selon un exemple, la deuxième interface de fixation du support d'accessoire constitue l'élément femelle.

Selon un exemple, la deuxième interface de fixation est configurée pour s'appairer mécaniquement de façon amovible avec ladite troisième interface de fixation par un appairage mécanique comprenant successivement une insertion de l'élément mâle dans l'élément femelle selon une direction d'insertion et une rotation de l'accessoire de sonde relativement au support d'accessoire selon un axe de rotation correspondant à la direction d'insertion.

Selon un exemple, l'élément femelle comprend :
- au moins une encoche d'insertion configurée pour que s'y insère un membre de verrouillage respectif dont est pourvu l'élément mâle ; et
- une glissière de positionnement débouchant respectivement sur chaque encoche d'insertion pour permettre un déplacement, par rotation de l'accessoire de sonde relativement au support d'accessoire, du membre de verrouillage respectif dans la glissière de positionnement jusqu'à une position de verrouillage causant ainsi l'appairage mécanique du support d'accessoire avec l'accessoire de sonde.

Selon un exemple, l'une parmi les deuxième et troisième interfaces de fixation comprend au moins une ailette déformable configurée pour se déformer en réponse au déplacement du membre de verrouillage respectif dans la glissière de verrouillage jusqu'à la position de verrouillage de sorte à causer une force de friction s'opposant audit déplacement.

Selon un exemple, ladite au moins une ailette déformable est comprise dans la deuxième interface de fixation du support d'accessoire.

Selon un exemple, la deuxième interface de fixation comprend deux ailettes déformables.

Selon un exemple, l'une parmi les deuxième et troisième interfaces de fixation comprend au moins une première cavité, ladite au moins une ailette déformable et ladite au moins une première cavité étant configurées pour que chaque ailette déformable s'engage dans une première cavité lorsque ledit au moins un membre de verrouillage atteint la position de verrouillage.

Selon un exemple, la deuxième interface de fixation comprend au moins deux encoches d'insertion de taille différente configurées pour que s'y engagent des membres de verrouillage respectifs de la troisième interface de fixation.

Selon un exemple, l'une parmi les deuxième et troisième interfaces de fixation comprend une deuxième cavité et l'autre comprend une protubérance configurée pour s'engager dans la deuxième cavité lorsque ledit au moins un membre de verrouillage atteint la position de verrouillage.

Selon un exemple, les deuxième et troisième interfaces de fixation sont configurées pour être appairées mécaniquement de façon amovible par une rotation de 1/4 de tour du support d'accessoire relativement à l'accessoire de sonde.

Selon un deuxième aspect, la présente divulgation vise un support d'accessoire pour sonde ultrasonore, ce support d'accessoire comprenant :
- un support d'accessoire tel que défini selon le premier aspect de la présente divulgation; et
- un accessoire de sonde comprenant une troisième interface de fixation de forme complémentaire à la deuxième interface de fixation du support d'accessoire,
dans lequel les deuxième et troisième interfaces de fixation sont configurées pour s'appairer mécaniquement de façon amovible par rotation de l'accessoire de sonde relativement au support d'accessoire.

Le système accessoire selon la divulgation peut comporter d'autres caractéristiques qui peuvent être prises séparément ou en combinaison, notamment parmi les modes de réalisation qui suivent qui sont présentés à titre d'illustration uniquement et peuvent être combinés ou associés sauf stipulation contraire.

Selon un exemple, l'une parmi les deuxième et troisième interfaces de fixation constitue un élément mâle et l'autre constitue un élément femelle, lesdits éléments mâle et femelle étant configurés pour s'appairer mécaniquement en rotation par insertion de l'élément mâle dans l'élément femelle.

Selon un exemple, les deuxième et troisième interfaces de fixation sont configurées pour s'appairer mécaniquement de façon amovible par un appairage mécanique comprenant successivement une insertion de l'élément mâle dans l'élément femelle selon une direction d'insertion et une rotation de l'accessoire de sonde relativement au support d'accessoire selon un axe de rotation correspondant à la direction d'insertion.

Selon un exemple, l'accessoire de sonde comprend au moins l'un parmi :
- un capteur ;
- un distributeur de gel ; et
- une source lumineuse.

Selon un exemple, l'accessoire de sonde comprend un capteur de position.

Selon un exemple, l'accessoire de sonde forme un bouton rotatif.

Selon un troisième aspect, la présente divulgation concerne un procédé de fixation du système accessoire tel que défini dans le deuxième aspect de la présente divulgation. Ce procédé de fixation comprend :
- insertion d'un membre de verrouillage de l'élément mâle dans respectivement une encoche d'insertion de l'élément femelle ; et
- déplacement, par rotation de l'accessoire de sonde relativement au support d'accessoire, du membre de verrouillage dans, respectivement, la glissière de positionnement jusqu'à une position de verrouillage causant ainsi l'appairage mécanique du support d'accessoire avec l'accessoire de sonde.

La présente divulgation permet ainsi avantageusement d'offrir un support d'accessoire pour sonde ultrasonore assurant une fixation amovible d'un accessoire à une sonde ultrasonore, cette fixation étant fiable et robuste tout en garantissant une bonne ergonomie d'utilisation et un nettoyage aisé, efficace et rapide.

Les caractéristiques et avantages de la divulgation apparaitront plus précisément à la lecture de la description qui va suivre, donnée uniquement à titre d'exemple non limitatif, et faite en référence aux figures annexées. Notamment, les exemples illustrés dans les figures peuvent être combinés entre eux, sauf incohérence mentionnée ou notoire.

### Brève description des figures

D'autres caractéristiques et avantages de la présente divulgation ressortiront de la description des exemples de réalisation non limitatifs de la présente divulgation ci-après, en référence aux figures 1 à 15 annexées, sur lesquelles :
[Fig. 1] illustre schématiquement un système à ultrasons comprenant une sonde ultrasonore, selon un exemple particulier ;
[Fig. 2] illustre schématiquement un support d'accessoire et un système accessoire pour une sonde à ultrasons, selon un exemple de réalisation de la présente divulgation ;
[Fig. 3] illustre schématiquement le support d'accessoire et le système accessoire de la figure 2, selon un exemple de réalisation de la présente divulgation ;
[Fig. 4] illustre schématiquement un support d'accessoire et un système accessoire, selon un exemple de réalisation de la présente divulgation ;
[Fig. 5] est une vue en perspective éclatée représentant schématiquement un système accessoire comprenant un support d'accessoire et un accessoire pour sonde à ultrasons, selon un exemple de réalisation de la présente divulgation ;
[Fig. 6] illustre schématiquement un appairage mécanique d'un accessoire de sonde à une support d'accessoire (position P1), selon un exemple de réalisation de la présente divulgation ;
[Fig. 7] illustre schématiquement un appairage mécanique d'un accessoire de sonde à une support d'accessoire (position P2), selon un exemple de réalisation de la présente divulgation ;
[Fig. 8] représente schématiquement un procédé de fixation d'un système accessoire pour sonde à ultrasons, selon un exemple de réalisation de la présente divulgation ;
[Fig. 9] illustre schématiquement une interface de fixation d'un support d'accessoire pour sonde à ultrasons, selon un exemple de réalisation de la présente divulgation ;
[Fig. 10] est une vue en coupe représentant schématiquement l'interface de fixation du support d'accessoire de la figure 9, selon un exemple de réalisation de la présente divulgation ;
[Fig. 11] est une vue en perspective représentant schématiquement l'interface de fixation du support d'accessoire selon les figures 9 et 10, selon un exemple de réalisation de la présente divulgation ;
[Fig. 12] illustre schématiquement une interface de fixation d'un accessoire de sonde, selon un exemple de réalisation de la présente divulgation ;
[Fig. 13] illustre schématiquement des vues en perspective et de dessous de l'interface de fixation de l'accessoire de sonde de la figure 12, selon un exemple de réalisation de la présente divulgation ;
[Fig. 14] illustre schématiquement un support d'accessoire et un système accessoire pour une sonde à ultrason, selon un exemple de réalisation de la présente divulgation ; et
[Fig. 15] est une vue représentant une sonde ultrasonore équipée d'un support d'accessoire et d'un accessoire de sonde, selon un exemple de réalisation de la présente divulgation.

### Description des modes de réalisation

Une sonde ultrasonore, dite aussi sonde à ultrasons ou dispositif ultrasonore ou dispositif transducteur, est un dispositif émetteur et/ou récepteur d'ondes ultrasonores, pilotable au moyen de signaux électriques, ces signaux étant par exemple transmis entre la sonde ultrasonore et une unité de commande d'un système de commande.

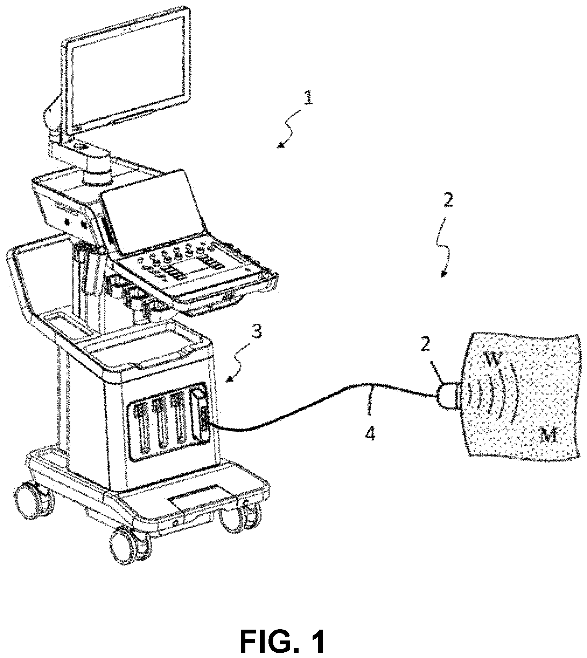

La figure 1 représente schématiquement, à titre purement illustratif, un exemple de réalisation d'un système ultrasonore 1 comprenant une sonde ultrasonore 2. Cette sonde 2 peut comprendre un ou une pluralité d'éléments transducteurs (par exemple un ou des éléments piézoélectriques) configurés pour émettre et/ou recevoir des ondes ultrasonores W. Chaque élément transducteur est ainsi configuré pour convertir un signal électrique reçu du système 1 en ondes ultrasonores et/ou réciproquement. La nature de ces ondes dépend du système 1 et/ou de la sonde 2, au vu notamment de l'utilisation qui en est faite et des objectifs à atteindre.

Les éléments transducteurs peuvent ainsi être configurés pour transmettre (a) des ondes (ou impulsions) ultrasonores W dans le milieu M et/ou pour recevoir (b) une pluralité de signaux ultrasonores W du milieu M, éventuellement en réponse à la transmission (a) des ondes W.

Selon un exemple, la sonde ultrasonore 2 est configurée pour émettre des ondes ultrasonores W. Selon un autre exemple, la sonde ultrasonore 2 est configurée pour recevoir des ondes ultrasonores W. Selon un autre exemple, la sonde ultrasonore 2 est configuré pour à la fois émettre et recevoir des ondes ultrasonores W.

Les éléments transducteurs de la sonde 2 peuvent comprendre des cristaux piézoélectriques et/ou d'autres composants qui peuvent être configurés pour générer et/ou enregistrer et/ou recevoir des signaux.

Comme illustré, le système ultrasonore 1 peut comprendre une unité (ou dispositif) de traitement 3 configurée pour piloter la sonde ultrasonore 2 au moyen de signaux électriques qui sont échangés (ou transmis) entre le système 1 et la sonde à ultrasons 2.

L'unité de traitement 3 est configurée pour générer des signaux électriques qui sont transmis à la sonde ultrasonore 2 pour causer l'émission, par ladite sonde, d'ondes ultrasonores W en direction et/ou dans un milieu M. Les signaux électriques ainsi générés sont représentatifs des (ou définissent les) ondes ultrasonores émis dans le milieu M.

L'unité de traitement 3 peut également être configurée pour traiter (ou interpréter) des signaux électriques reçus en provenance de la sonde ultrasonore 2. Ces signaux sont représentatifs d'ondes ultrasonores reçues par la sonde 2 en provenance du milieu M. Ces ondes forment par exemple un ou plusieurs échos ultrasonores, c'est-à-dire une réponse du milieu M aux ondes ultrasonores préalablement émises.

Le système 1 peut comprendre la sonde ultrasonore 2. En variante, la sonde ultrasonore 2 peut être externe au système 1. Par exemple, la sonde ultrasonore 2 peut être connecté au système 1 par un câble 4 (figure 1) ou peut communiquer sans fil avec lui.

Le système 1 peut être un système d'imagerie, par exemple dans le domaine médical. Les images générées par le système 1 peuvent être soit analysées en temps réel, par exemple par un utilisateur, soit analysées ultérieurement et/ou dans un autre lieu que celui dans lequel se trouve le système 1.

Le système 1 peut être un système médical, par exemple un système à ultrasons. En conséquence, la sonde ultrasonore 2 est par exemple une sonde échographique.

Par exemple, le système 1 peut être associé à une sonde à ultrasons 2, afin d'étudier un milieu M, notamment de collecter des données ultrasonores d'un tel milieu M. Le milieu M ainsi observé peut être de diverses natures selon le cas. Il peut s'agir par exemple de tissus vivants et/ou en particulier des tissus humains. L'observation d'un milieu M comprenant une ou des structures minérales par exemple est également possible (graviers, volcan, etc.).

Selon d'autres exemples, le système 1 et/ou la sonde ultrasonore 2 sont configurés à des fins de communication, d'imagerie ou de balayage, par exemple dans le domaine de l'imagerie médicale, du radar, du sonar, de la sismologie, des communications sans fil, de la radioastronomie, de l'acoustique et de la biomédecine.

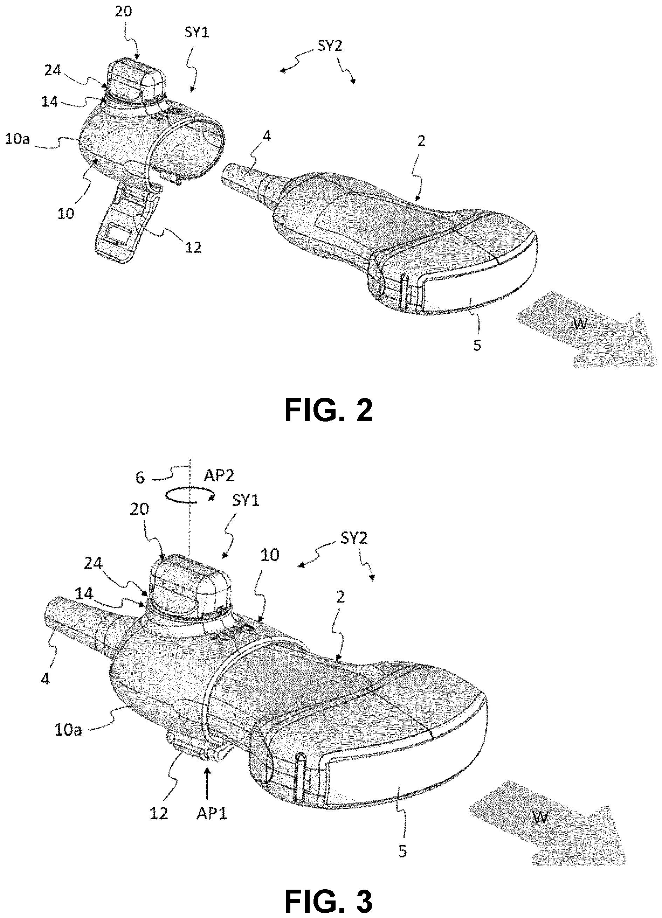

Des exemples de mise en oeuvre d'un support d'accessoire 10 pour sonde ultrasonore, et d'un système accessoire SY1 comprenant un tel support 10, ainsi qu'un procédé de fixation correspondant, vont maintenant être décrits dans ce qui va suivre en référence conjointement aux figures 2-15 fournies à titre d'illustration.

Sauf indications contraires, les éléments communs ou analogues à plusieurs figures portent les mêmes signes de référence et présentent des caractéristiques identiques ou analogues, de sorte que ces éléments communs ne sont généralement pas à nouveau décrits par souci de simplicité.

Les termes « premier(s) » (ou première(s)), « deuxième(s) », etc.) sont utilisés dans ce document par convention arbitraire pour permettre d'identifier et de distinguer différents éléments (tels que des opérations, des dispositifs, etc.) mis en oeuvre dans les modes de réalisation décrits ci-après.

Dans les exemples qui suivent, on considère à titre purement illustratif un support d'accessoire 10, et plus généralement un système accessoire SY1, configurés pour être fixés à une sonde ultrasonore 2 telle que précédemment décrite en référence à la figure 1. Comme cela apparaît au vu des figures 2-15, la configuration du support d'accessoire 10 et du système accessoire SY1, en termes notamment de forme, taille, etc., peut être adaptée selon le cas, et notamment en fonction de la sonde ultrasonore 2 considérée et/ou de l'accessoire 20.

Le support d'accessoire 10 est un support pour sonde ultrasonore, à savoir pour la sonde ultrasonore 2 dans les exemples considérés, ce support étant configuré pour supporter ou fixer, de façon amovible, un accessoire 20 (dit aussi accessoire de sonde) à la sonde ultrasonore 2. La nature et la configuration de cet accessoire 20 peuvent varier selon le cas. A titre purement illustratif, on considère dans les exemples qui suivent que l'accessoire de sonde 20 est un deuxième support, dit support de dispositif, configuré pour contenir ou supporter un dispositif accessoire, à savoir un capteur par exemple. Des variantes de cet accessoire 20 sont décrites par la suite.

Le système accessoire SY1 (figures 2-3) comprend le support d'accessoire 10 et l'accessoire 20, ces deux éléments 10 et 20 pouvant être fixées (ou attachés) ensemble de façon amovible comme décrit ci-après. L'accessoire 20 est un élément distinct du support d'accessoire 10.

Le système accessoire SY1 et la sonde ultrasonore 2 forment ensemble un système de sonde SY2 (figures 2-3) dans cet exemple.

Plus précisément, comme illustré notamment en figures 2-4, le support d'accessoire 10 comprend deux interfaces de fixation (dites aussi interfaces de verrouillage, ou dispositifs d'attache), à savoir une première interface de fixation 12 et une deuxième interface de fixation 14.

La première interface de fixation 12 est configurée pour s'appairer (ou être appariée) mécaniquement à la sonde ultrasonore 2. Autrement dit, cette interface de fixation 12 est conçue pour coupler (ou fixer) ensemble mécaniquement la sonde ultrasonore 2 et le support d'accessoire 10. Le support d'accessoire 20 est donc un élément externe à (ou distinct de) la sonde ultrasonore 2. L'interface de fixation 12 peut comprendre un quelconque mécanisme de fixation permettant d'ancrer le support d'accessoire 10 solidairement à la sonde 2.

Dans les exemples décrits, l'interface de fixation 12 est configurée pour appairer mécaniquement (ou associer ou fixer) de façon amovible le support d'accessoire 10, et plus généralement le système accessoire SY1, à la sonde ultrasonore 2. L'appairage (ou couplage) mécanique du support d'accessoire 10 à la sonde 2 est noté AP1 (figure 3). Une fois l'appairage AP1 réalisé, un utilisateur peut donc si besoin détacher le support d'accessoire 10 de la sonde ultrasonore 2 par une opération de découplage (ou détachement) inverse à l'opération d'appairage AP1. Des variantes dans lesquelles l'appairage mécanique AP1 du support d'accessoire 10 sur la sonde 2 est permanent sont toutefois possibles.

Comme illustré en figures 2-4, le support d'accessoire 10 peut être configuré pour que s'y insère la sonde ultrasonore 2. Pour ce faire, le support d'accessoire 10 peut comprendre une surface interne de forme complémentaire à la forme d'au moins une partie de la sonde ultrasonore 2. Le support d'accessoire 10 forme par exemple une cavité configurée pour que puisse y être insérée tout ou partie de la sonde ultrasonore 2.

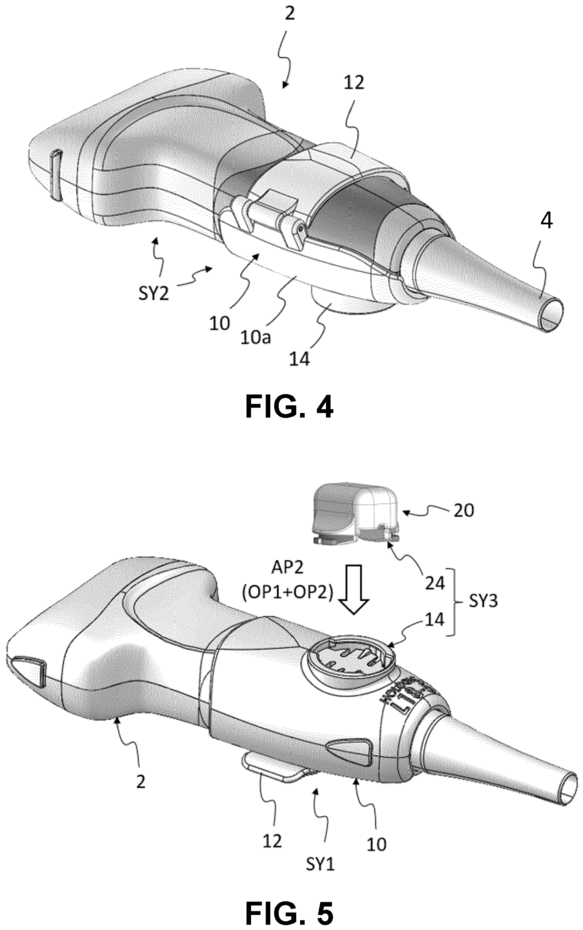

Selon un exemple, la première interface de fixation 12 comprend un bras articulé qui est monté en rotation sur le corps 10a du support d'accessoire 10. Une fois la sonde ultrasonore 2 insérée dans le support d'accessoire 10, le bras articulé peut être déplacé en rotation depuis une position ouverte dans une position fermée (et inversement), causant ainsi l'appairage mécanique AP1 du support d'accessoire 10 sur la sonde ultrasonore 2 (et inversement le découplage mécanique de ces éléments). La configuration de ce bras peut varier selon le cas et selon le type de sonde utilisé. Un exemple de mise en oeuvre de ce bras articulé est représenté en figure 4.

La deuxième interface de fixation 14 du support d'accessoire 10 est de forme complémentaire à une troisième interface de fixation 24 (figures 2-3) dont est équipé (ou pourvu) l'accessoire de sonde 20. Autrement dit, l'accessoire 20 comprend la troisième interface de fixation 24 de forme complémentaire à celle de la deuxième interface de fixation 14. L'interface de fixation 14 du support d'accessoire 10 est ainsi configurée pour s'appairer mécaniquement de façon amovible (ou réversible) avec la troisième interface de fixation 24 par rotation de l'accessoire de sonde 20 relativement au support d'accessoire 10. L'appairage (ou couplage) mécanique du support d'accessoire 10 avec l'accessoire 20 est noté AP2 (figure 3).

Les deuxième et troisième interfaces de fixation 14 et 24 forment ensemble un système de fixation SY3 (figure 5).

Leur complémentarité mutuelle de forme permet aux interfaces de fixation 14 et 24 de coopérer ensemble mécaniquement pour permettre une fixation, de façon amovible (ou réversible), de l'accessoire 20 au support d'accessoire 10. Une fois l'appairage AP2 réalisé, un utilisateur peut donc si besoin détacher (ou découpler, ou désolidariser) l'accessoire 20 vis-à-vis du support d'accessoire 10 par une opération de découplage (ou détachement) inverse à l'opération d'appairage AP2. Le caractère réversible (ou amovible) de l'appairage AP2 signifie qu'un utilisateur peut sélectivement attacher et détacher les interfaces de fixation 14 et 24 l'une par rapport à l'autre.

La complémentarité de forme entre les interfaces de fixation 14 et 24 peut être assurée de diverses manières. Selon un exemple, l'une parmi les deuxième et troisième interfaces de fixation 14 et 24 constitue un élément mâle et l'autre constitue un élément femelle, ces éléments mâle et femelle étant configurés pour s'appairer (se coupler) mécaniquement en rotation par insertion de l'élément mâle dans l'élément femelle.

Comme illustré notamment en figures 2-5, on suppose par la suite à titre purement illustratif que la troisième interface de fixation 24 constitue l'élément mâle et que la deuxième interface de fixation 14 constitue l'élément femelle. Ainsi, les interfaces de fixation 14 et 24 sont configurées pour s'appairer mécaniquement en rotation par insertion de (tout ou partie) de l'interface de fixation 24 dans (tout ou partie) de l'interface de fixation 14.

Des variantes selon la configuration inverse sont toutefois possibles. Ainsi, les exemples de réalisation décrits dans la présente divulgation peuvent s'appliquer de façon analogue au cas alternatif où la deuxième interface de fixation 14 constitue l'élément mâle et la troisième interface de fixation 24 constitue l'élément femelle.

La configuration de la deuxième interface de fixation 14 en tant qu'élément femelle permet avantageusement d'obtenir un appairage mécanique AP2 plus robuste que dans la configuration inverse, en particulier si la sonde ultrasonore 2 est considérée comme une partie fixe et l'accessoire 20 comme une partie mobile. L'accessoire 20 peut par exemple être qualifiée de partie mobile si cette partie est saisie manuellement et déplacée par l'utilisateur au cours des opérations OP1 et OP2 tandis que le support d'accessoire 20 reste substantiellement immobile dans l'espace.

A noter que la rotation relative OP1 de l'accessoire 20 par rapport au support d'accessoire 10 peut être réalisée de diverses manières, par exemple en opérant une rotation de l'accessoire 20 tout en maintenant à une position fixe le support d'accessoire 10, ou en opérant une rotation du support d'accessoire 10 tout en maintenant à une position fixe l'accessoire 20, ou encore en opérant des rotations (de sens opposés) à la fois du support d'accessoire 10 et de l'accessoire 20.

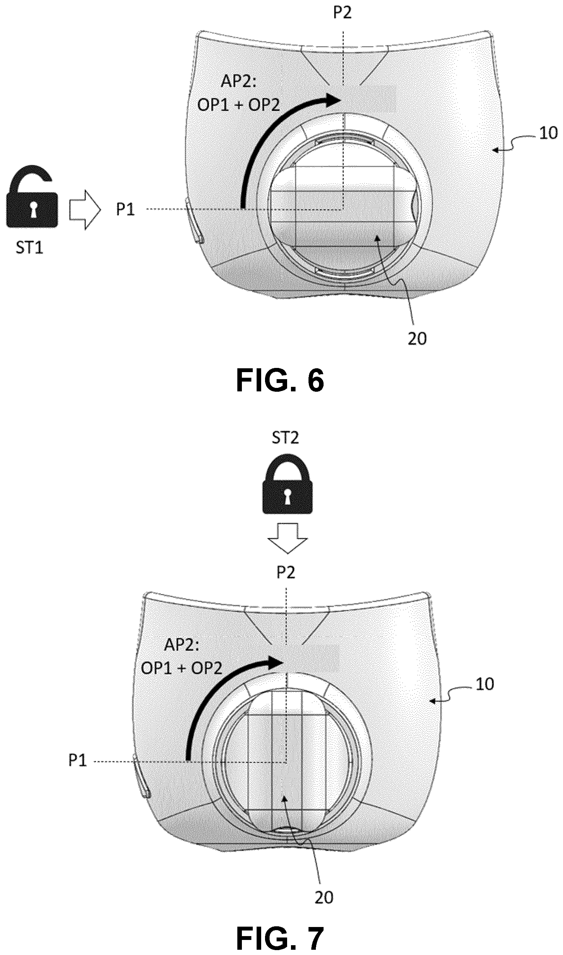

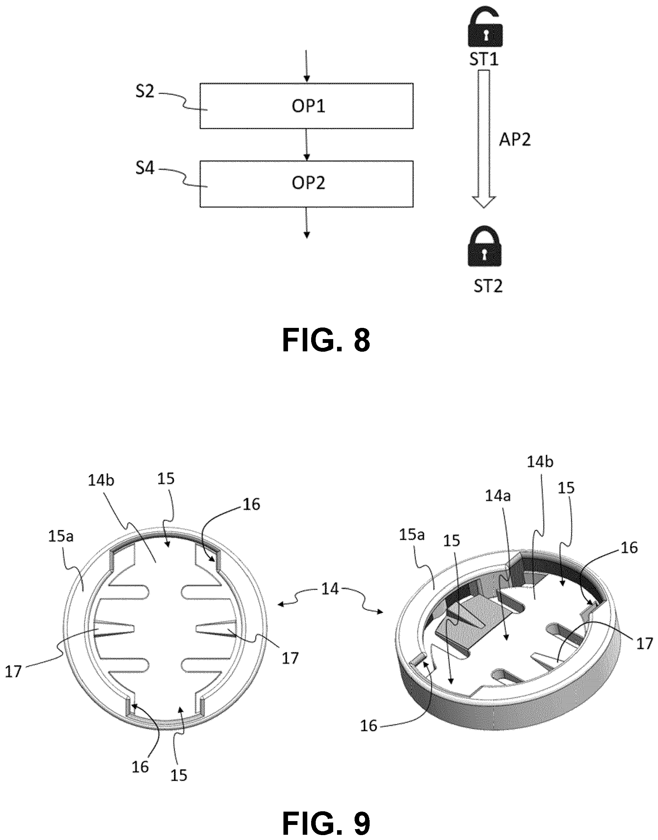

Les figures 6-7 représentent le processus d'appairage mécanique AP2 de l'accessoire 20 avec le support d'accessoire 10 selon un exemple particulier. La figure 8 représente sous forme d'un diagramme les étapes d'un procédé de fixation visant à fixer ensemble le support d'accessoire 10 et l'accessoire 20 selon l'appairage mécanique AP2.

Comme illustré à titre d'exemple en figures 3-8, l'appairage mécanique AP2 est accompli par réalisation successive d'une opération OP1 d'insertion et d'une opération OP2 de rotation. On suppose à un stade initial que les interfaces de fixation 12 et 24 se trouvent dans un premier état ST1 non appairé. Au cours de l'opération OP1 d'insertion (étape S2, figure 8), l'interface de fixation 24 de l'accessoire 20 est inséré (ou engagé) OP1 dans l'interface de fixation 14 du support d'accessoire 10 (par exemple par un mouvement de translation). Une fois l'interface de fixation 24 insérée en position dans l'interface de fixation 14, une rotation OP2 de l'accessoire 20 relativement au support d'accessoire 10 est opérée selon un axe de rotation AX1 (étape S4, figure 8) depuis une première position angulaire P1 dans une deuxième position angulaire P2 dite position de verrouillage, causant ainsi l'appairage mécanique AP2 de l'accessoire 20 avec le support d'accessoire 10. L'appairage mécanique AP2 permet ainsi de passer les interfaces de fixation 14 et 24 depuis l'état ST1 non appairé à un deuxième état ST2 appairé (position P2).

Les manières dont sont réalisées les opérations (ou mouvements) d'insertion OP1 et de rotation OP2 peuvent varier selon les configurations des interfaces de fixation 12 et 14. Selon un exemple illustré notamment en figure 3 et 5-8, la deuxième interface de fixation 14 est configurée pour s'appairer mécaniquement de façon amovible avec la troisième interface de fixation 24 par un appairage mécanique AP2 comprenant successivement une insertion OP1 de l'élément mâle (à savoir la troisième interface de fixation 24 dans cet exemple) dans l'élément femelle (à savoir la deuxième interface de fixation 14 dans cet exemple) selon une direction d'insertion 6 et une rotation OP2 de l'accessoire de sonde 20 relativement au support d'accessoire 10 selon un axe de rotation correspondant à la direction d'insertion 6. Autrement dit, une fois que l'élément mâle est inséré (ou engagé) dans l'élément femelle par un mouvement d'insertion OP1 (par translation) selon l'axe 6, une rotation OP2 de l'accessoire de sonde 20 relativement au support d'accessoire 10 est opérée selon la direction (ou l'axe) 6 servant d'axe de rotation. L'appairage AP2 est ainsi réalisé par exécution successive des opérations OP1 et OP2. De cette manière, on peut avantageusement coupler mécaniquement de façon amovible les deuxième et troisième interfaces de fixation 14 et 24 par un mouvement continu selon une même direction 6, à savoir une translation selon l'axe 6 puis une rotation autour de ce même axe 6. Cette correspondance de direction entre mouvement d'insertion OP1 et mouvement de rotation OP2 permet par exemple avantageusement à un utilisateur d'appairer aisément et rapidement le support d'accessoire 10 avec l'accessoire de sonde 20 par un mouvement manuel continu comprenant successivement l'insertion OP1 puis la rotation OP2, et ce avec précision et un minimum de manipulation.

On outre, le couplage selon la présente divulgation permet de tenir efficacement l'accessoire de sonde, quel que soit son poids, y compris dans des cas où son poids est supérieur, voire substantiellement supérieur, à celui de la sonde. En effet, dans des cas particuliers où l'accessoire de sonde est relativement lourd par rapport à la sonde, un risque pourrait éventuellement se poser dans des systèmes de couplage traditionnels en ce que l'accessoire pourrait causer un mouvement involontaire par l'action de son poids et ainsi causer un découplage intempestif de l'ensemble. Grâce au système de la présente divulgation, les forces inertielles et moments susceptibles d'être appliqués par l'accessoire de sonde sur le mécanisme de fixation (les interfaces de fixation) du support de sonde ne s'appliquent généralement pas selon une direction (par exemple, pas selon l'axe 6) qui pourrait causer un découplage intempestif, lié notamment au poids de l'accessoire, ce qui permet avantageusement de sécuriser le couplage.

On considère à titre purement illustratif que les interfaces de fixation 14 et 24 sont configurées pour que la rotation OP1 (S4) soit réalisée selon le sens horaire (comme illustré en figures 3 et 6-7). Selon une variante, la rotation OP1 est réalisée selon le sens anti-horaire.

Selon un exemple, les deuxième et troisième interfaces de fixation 14 et 24 sont configurées pour être appairées mécaniquement ensemble de façon amovible (ou réversible) par une rotation OP2 de 1/n tour du support d'accessoire 10 relativement à l'accessoire de sonde 20, où n est un entier compris entre 2 et 4.

Selon un exemple, n = 4. Autrement dit, les deuxième et troisième interfaces de fixation 14 et 24 peuvent être configurées pour être appairées mécaniquement ensemble de façon amovible par une rotation OP2 de ¼ de tour du support d'accessoire 10 relativement à l'accessoire de sonde 20 (figures 6-7). Cette configuration particulière est avantageuse en ce qu'elle permet d'obtenir un bon compromis entre rapidité d'exécution de l'appairage mécanique AP2 par un utilisateur et robustesse de l'appairage AP2.

Comme illustré dans les exemples des figures 2-5, la sonde ultrasonore 2 comprend une sortie d'émission (ou partie émettrice) 5, située à une extrémité distale de la sonde, configurée pour émettre des ondes ultrasonores W. Le support d'accessoire 10 est configuré pour que, une fois l'appairage mécanique AP1 réalisé, cette sortie d'émission 5 est découverte ou libre (c'est-à-dire non couverte par le support d'accessoire 10), ce qui permet à des ondes ultrasonores W d'être émises depuis cette sortie 5. Selon un exemple, le support d'accessoire 20 comprendre une ouverture positionnée en correspondance avec la sortie d'émission 5 pour découvrir cette dernière lorsque l'appairage mécanique AP1 du support d'accessoire 10 avec la sonde 2 est réalisé.

La conception des interfaces de fixation 14 et 24 peut être adaptée selon le cas. Des exemples de mises en oeuvre de ces interfaces de fixation sont à présent décrits en référence aux figures 9-13.

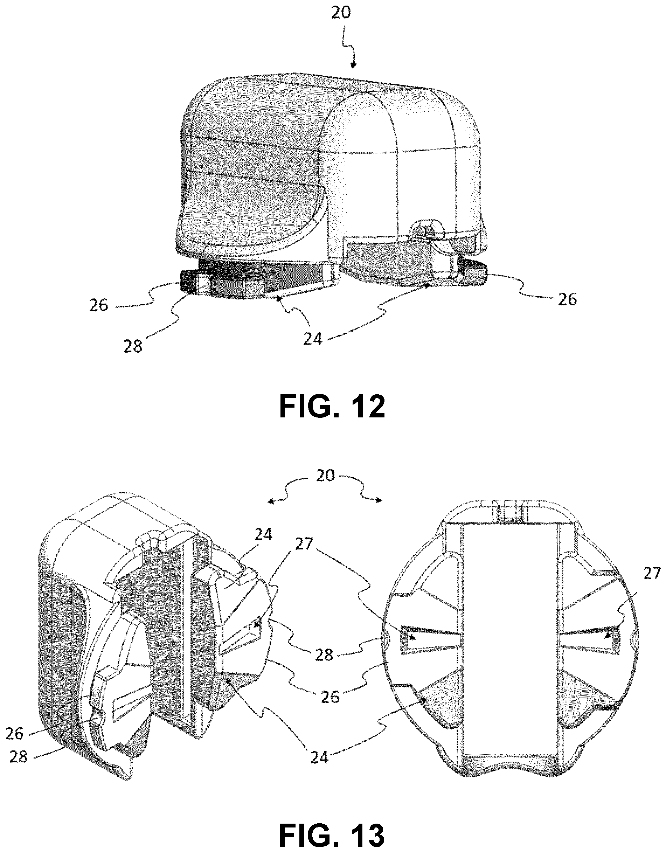

Dans les exemples qui suivent, on suppose que l'accessoire 20 comprend au moins un membre de verrouillage 26 (figures 12-13) configuré pour permettre le verrouillage de l'interface de fixation 24 avec l'interface de fixation 14 lors de l'appairage mécanique AP2. D'autres variantes sont envisageables comme indiqué ci-après.

Selon un exemple illustré en figures 9-11, l'élément femelle, à savoir l'interface de fixation 14 dans cet exemple, comprend au moins une encoche d'insertion 15 et une glissière de positionnement 16 associée respectivement à chaque encoche d'insertion 15. Chaque encoche d'insertion 15 est configurée pour que s'y insère un membre de verrouillage 26 respectif dont est pourvu l'accessoire 20. En outre, une glissière de positionnement 16 débouche respectivement sur chaque encoche d'insertion 15 pour permettre un déplacement, par rotation OP2 de l'accessoire de sonde 20 relativement au support d'accessoire 10, du membre de verrouillage 26 respectif dans la glissière de positionnement 15 jusqu'à une position de verrouillage P2, ce déplacement causant ainsi l'appairage mécanique AP2 du support d'accessoire 10 avec l'accessoire de sonde 20.

Des variantes selon une configuration inverse sont également possibles, variantes dans lesquelles l'interface de fixation 24 de l'accessoire 20 comprend la ou les encoches d'insertion 16 et une glissière de positionnement 16 associée respectivement à chaque encoche d'insertion 15 et dans lesquelles l'interface de fixation 14 du support d'accessoire 10 comprend la ou les membres de verrouillage 26 correspondants. Aussi, les divers exemples décrits dans cette divulgation s'appliquent de façon analogue à cette configuration inverse.

Comme illustré en figures 9-13, on suppose dans les exemples qui suivent à titre purement illustratif que l'accessoire 20 comprend deux membres de verrouillage 26. A cet effet, l'interface de fixation 14 du support d'accessoire 10 comprend donc deux encoches d'insertion 15 et deux glissières de positionnement 16 débouchant respectivement sur ces encoches d'insertion 15 pour permettre un déplacement, par rotation OP2 de l'accessoire de sonde 20 relativement au support d'accessoire 10, des membres de verrouillage 26 dans leur glissière de positionnement respective 15 jusqu'à une position de verrouillage P2, ce déplacement causant ainsi l'appairage mécanique AP2 du support d'accessoire 10 avec l'accessoire de sonde 20.

A noter toutefois que des variantes avec un nombre différent de membres de verrouillage 26, et donc d'encoches d'insertion 15 et de glissières de positionnement 16 associées, sont possibles. A titre d'exemple, l'interface de fixation 24 de l'accessoire 20 peut comprendre un unique membre de verrouillage 26. Dans ce cas, l'interface de fixation 14 du support d'accessoire 10 peut comprendre une unique encoche d'insertion 15 ainsi qu'une unique glissière de positionnement 16 associée pour permettre le déplacement de l'unique membre de verrouillage 26 jusqu'à la position de verrouillage P2. En variante, des mises en oeuvre avec plus de deux membres de verrouillage 26, et autant d'encoches d'insertion 15 et de glissières de positionnement 16, sont possibles. L'utilisation de deux membres de verrouillage 26 ou plus permet avantageusement de rendre l'appairage mécanique AP2 robuste tout en limitant sa complexité de fabrication.

Ainsi, dans l'exemple considéré (figures 8-13), au cours de l'étape S2 d'insertion, l'opération d'insertion OP1 cause l'insertion de chacun des deux membres de verrouillage 26 dans une encoche d'insertion 15 correspondante prévue à cet effet dans l'interface de fixation 14 du support d'accessoire 10. Une fois les membres de verrouillage 26 insérés en position dans l'interface de fixation 14, la rotation OP2 de l'accessoire 20 relativement au support d'accessoire 10 est opérée selon l'axe de rotation AX1 depuis la première position angulaire P1 jusque dans la deuxième position angulaire P2 (position de verrouillage), causant ainsi l'appairage mécanique AP2 de l'accessoire 20 avec le support d'accessoire 10. Le déplacement des membres de verrouillage 26 depuis la position P1 à la position P2 le long de leur glissière de positionnement respective 16 permet ainsi d'appairer mécaniquement ensemble (AP2) les interfaces de fixation 14 et 24. Ainsi, lorsque les membres de verrouillage 26 sont positionnés selon la position P1, les interfaces de fixation 12 et 24 se trouvent dans le premier état ST1 non appairé. Lorsque les membres de verrouillage 26 sont positionnés selon la position P2, les interfaces de fixation 12 et 24 se trouvent dans le deuxième état ST2 appairé.

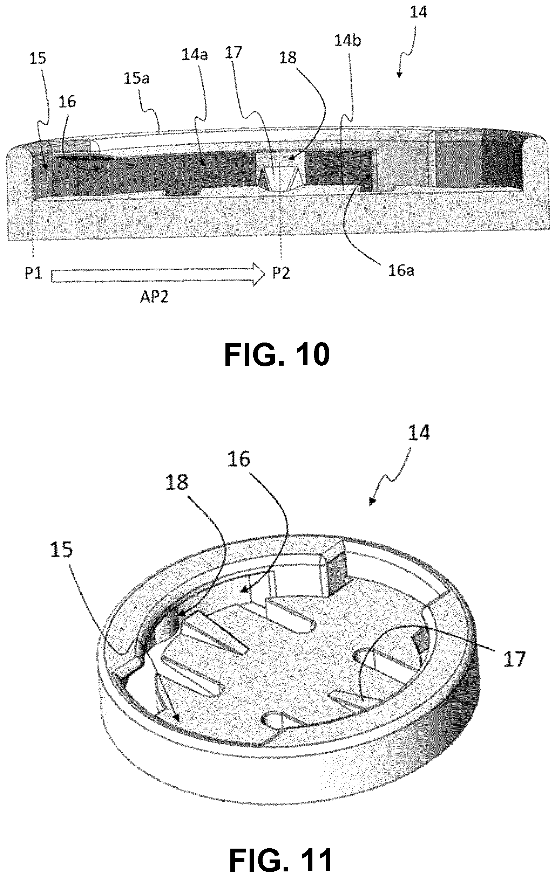

Comme illustré notamment en figures 9-11, l'interface de fixation 14 du support d'accessoire 10 peut comprendre une cavité 14a configurée pour recevoir au moins une partie de l'interface de fixation 24 de l'accessoire 20. Cette cavité 14a facilite l'insertion OP1 des membres de verrouillage 26 dans leurs encoches d'insertion 15 respectives. La ou les glissières de positionnement 16 peuvent alors être formées en bordure de cette cavité 14a pour permettre un déplacement aisé des membres de verrouillage 26 dans les glissières 16 par rotation OP2 de l'accessoire 20 relativement au support d'accessoire 30, selon l'axe de rotation AX1 (figures 3 et 10).

Dans les exemples considérés, les glissières de positionnement 16 sont chacune définies par au moins un bord supérieur 15a de glissière (figures 9-11). Les encoches d'insertion 15 sont alors chacune positionnées de sorte à définir l'entrée d'une glissière de positionnement 15 respective. Chaque membre de verrouillage 26 peut ainsi atteindre une glissière 16 respective au travers de l'encoche d'insertion 15 associée.

La configuration des membres de verrouillage 26 (notamment en termes de position, forme, nombre, etc.) peut être adaptée en fonction de celle des encoches d'insertion 15 et des glissières de positionnement 16, et réciproquement. Comme représenté à titre d'exemple en figures 12-13, les membres de verrouillage 26 peuvent être (ou comprendre) divers éléments rigides, présentant par exemple la forme d'ailettes, de protubérances ou de picots, aptes à s'introduire dans les encoches d'insertion 15 et à se déplacer par rotation OP2 dans les glissières de positionnement 16 jusqu'à leur position de verrouillage P2 respective.

Comme illustré en figures 9-11, chaque glissière de positionnement 16 peut être munie d'une butée 16a de fin de course, marquant la position de verrouillage P2 du membre de verrouillage 26 qu'elle reçoit. Ces butées 16a sont configurées de sorte à bloquer les membres de verrouillage 26 lorsqu'ils atteignent leur position de verrouillage P2 dans leur glissière de positionnement 16 respective en fin de rotation OP2. Grâce à ces butées 16a, le verrouillage de l'accessoire 20 à la position souhaitée relativement au support d'accessoire 10 peut être assuré.

Selon un exemple, la deuxième interface de fixation 14 comprend deux encoches d'insertion 15 (voire 3 ou plus), potentiellement de tailles différentes, ces encoches d'insertion étant configurées pour que s'y engagent (ou s'y insèrent) des membres de verrouillage 26 respectifs de la troisième interface de fixation 24 lors de l'opération d'insertion OP1 (étape S2, figure 8). Comme illustré en figures 9 et 11 par exemple, l'une des encoches d'insertion 15 est plus grande que l'autre. De même, l'un des membres de verrouillage 26 est plus grand que l'autre, chacun de ces membres de verrouillage 26 étant configuré pour être inséré dans une encoche d'insertion 15 associée. Cette configuration permet avantageusement de former un détrompeur au niveau de l'interface de fixation 14 pour éviter toute erreur de positionnement de l'accessoire 20 par rapport au support d'accessoire 10. Pour ce faire, l'un des deux membres de verrouillage 26 peut être configuré pour être plus grand (ou plus large) qu'une autre encoche d'insertion 15, à savoir dans cet exemple celle conçue pour recevoir l'autre membre de verrouillage 26. De cette manière, une seule orientation de l'accessoire 20 est possible relativement au support d'accessoire 10, ce qui permet d'éviter toute erreur de positionnement au moment de l'appairage mécanique AP2. Cette configuration peut être particulièrement avantageuse dans certains contextes d'utilisation, par exemple dans un contexte d'intervention chirurgicale où il convient de manipuler de façon efficace, rapide et fiable les différents éléments en jeu, et ce malgré un environnement contraignant (en raison par exemple du port de gants, perturbations sonores ou autres, stress, enjeux élevés en cas d'erreur, etc.).

Selon un exemple, les deux encoches d'insertion 15 de l'interface de fixation 14 sont positionnées de façon diamétralement opposées vis-à-vis de l'axe de rotation AX1 de l'accessoire 20 relativement au support d'accessoire 10. Cette configuration permet un appairage mécanique AP2 robuste et aisé pour l'utilisateur.

Selon un exemple, l'une parmi les deuxième et troisième interfaces de fixation 14 et 24 comprend au moins une ailette déformable 17 configurée pour se déformer en réponse au déplacement d'un membre de verrouillage respectif 26 dans sa glissière de verrouillage 16 jusqu'à sa position de verrouillage P2 de sorte à causer une force de friction s'opposant audit déplacement. Comme illustré en figures 9-11, on suppose à titre d'exemple que les ailettes déformables 17 sont au nombre de deux et sont comprises dans l'interface de fixation 14 du support d'accessoire 10. A noter que diverses configurations en termes notamment de nombre, de positionnement et de forme de ces ailettes déformables 17 sont possibles. De manière générale, au moins une ailette déformable 14 peut être disposée dans la deuxième interface de fixation 14, ou dans la troisième interface de fixation 24, ou dans les deux.

Plus précisément, dans les exemples illustrés en figures 9-11, les deux ailettes déformables 17 sont disposées au fond 14b de la cavité 14a ménagée dans l'interface de fixation 14 du support d'accessoire 10. Ces ailettes 17 sont configurées pour se déformer sous l'effet du déplacement des membres de verrouillage 26 depuis la position P1 à la position de verrouillage P2 lors de la rotation OP2, causant ainsi une force de friction s'opposant (contraire) au déplacement des membres de verrouillage 26. Cette force de friction résulte du frottement des ailettes déformables 17 contre une surface de contact de la troisième interface de fixation 24 (par exemple une surface de contact des membres de verrouillage 26). Pour ce faire, les ailettes déformables 17 peuvent être formée d'un matériau élastique (par exemple un polyamide (PA) ou de l'acier) choisi en fonction de ses propriétés élastiques et des besoins au cas par cas. Le matériau est choisi de préférence avec une limite élastique élevée pour garantir un effet ressort des ailettes déformables 17.

Il est avantageux de placer la ou les ailettes déformables 17 au niveau de l'interface de fixation 14 du support sonde 10 (plutôt qu'au niveau de l'interface de fixation 24) dans la mesure où cela permet d'obtenir un système de verrouillage SY3 plus robuste, en particulier si l'on considère que le support de sonde 10 (et plus généralement la sonde ultrasonore 2) constitue une partie fixe. En variante, la ou les ailettes déformables 17 peuvent toutefois être placées au niveau de l'interface de fixation 24 de l'accessoire 20.

Il est avantageux que les ailettes déformables 17 soient au nombre de deux ou plus, dans la mesure où cela rend le système de verrouillage SY3 plus robuste et plus facile à manipuler. Comme illustré, les ailettes déformables 17 peuvent être au nombre de deux, ce qui permet de limiter l'encombrement et de permettre un bon compromis entre les dimensions des ailettes et la force de maintien obtenue. Un tel compromis peut en particulier être avantageusement atteint en positionnant deux ailettes déformables 17 diamétralement opposée l'une de l'autre par rapport à l'axe de rotation AX1 de l'accessoire 20 relativement au support d'accessoire 10.

Selon un exemple illustré en figures 9-13, l'interface de fixation 24 comprend deux cavités 27 configurées pour que s'y engage une ailette déformable 17 respective lorsque les membres de verrouillage 26 atteignent leur position de verrouillage P2 (en fin de rotation OP2). Le ménagement de ces cavités 27 facilite le maintien en position de verrouillage P2 de l'accessoire de sonde 20 relativement au support d'accessoire 10. Pour ce faire, chaque cavité 27 peut présenter une forme complémentaire à celle de l'ailettes déformable 17 qu'elle reçoit.

A noter que la configuration de ces cavités 27, en termes notamment de nombre et de positionnement, peut être adaptée selon le cas en fonction de la configuration de la ou des ailettes déformables 27. De façon générale, au moins l'une parmi les deuxième et troisième interfaces de fixation 14 et 24 peut comprendre une telle cavité 27 (figure 13) configurée pour recevoir une ailette déformable 27 correspondante lorsque le ou les membres de verrouillage 26 atteignent leur position de verrouillage P2 (en fin de rotation OP2). En variante, une unique cavité 27 peut être ménagée dans l'interface de fixation 14 (et/ou dans l'interface de fixation 24) pour que s'y insère une unique ailette déformable 27 ménagée dans l'interface de fixation 24 (et/ou dans l'interface de fixation 14 respectivement). En variante, une pluralité de telles cavités 27 peuvent être ménagées dans l'interface de fixation 14 et/ou dans l'interface de fixation 24.

Selon un exemple illustré en figures 9-13, la deuxième interface de fixation 14 comprend deux protubérances 18 et la troisième interface de fixation 24 comprend deux cavités (ou renfoncements) 28 correspondants. Chaque protubérance 18 est configurée pour s'engager dans une cavité 28 respective lorsque les membres de verrouillages 26 atteignent leur position de verrouillage P2 en fin de rotation OP2. Pour ce faire, les protubérances 18 et les cavités 28 peuvent présenter des formes complémentaires pour coopérer mécaniquement ensemble selon des systèmes de type mâle-femelle. Le ménagement de telles protubérances 18 et de telles cavités 28 permet avantageusement d'obtenir un verrouillage plus robuste lors de l'appairage mécanique AP2.

Des variantes sont toutefois possibles, notamment en termes de nombre et de positionnement des protubérances 18 et des cavités 18. En variante, la ou les protubérances 18 peuvent être ménagées dans l'interface de fixation 24 et la ou les cavités 28 peuvent être ménagées dans l'interface de fixation 14. De façon plus générale, l'une parmi les deuxième et troisième interfaces de fixation 14 et 24 peut ainsi comprendre une cavité 28 et l'autre comprendre une protubérance 18 configurée pour s'engager dans la cavité 18 lorsque ledit au moins un membre de verrouillage 26 atteint la position de verrouillage P2.

Selon un exemple illustré en figures 9-13, chaque membre de verrouillage 26 de l'accessoire 20 comprend une cavité 28 respective, et réciproquement chaque glissière de positionnement 16 de l'interface de fixation 14 comprend une protubérance 18 configurée pour s'engager dans une cavité 28 respective lorsque les membres de verrouillages 26 atteignent leur position de verrouillage P2 en fin de rotation OP2. Cela permet avantageusement d'obtenir un verrouillage particulièrement robuste lors de l'appairage mécanique AP2.

A noter que diverses configurations du support d'accessoire 10 et de l'accessoire de sonde 20 sont possibles, notamment en termes de forme, taille, dimensions, etc. La configuration du support d'accessoire 10 et de l'accessoire de sonde 20 peuvent en particulier être adaptée en fonction de la configuration de la sonde à ultrasons 2.

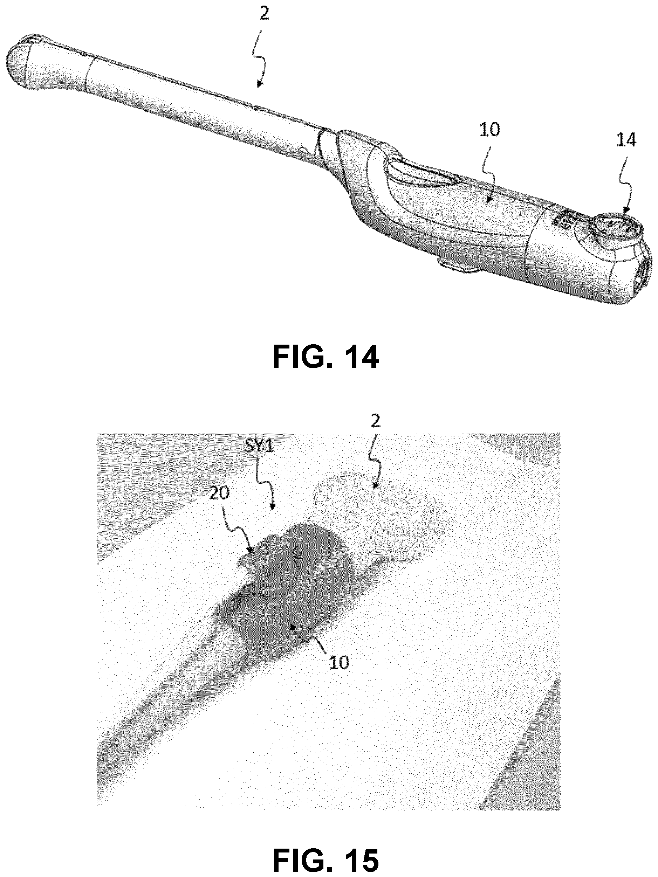

Les figures 14 et 15 représentent deux variantes de réalisation du support d'accessoire 10 et de l'accessoire de sonde 20, dont les configurations respectives sont adaptées en fonction de la configuration de la sonde ultrasonore 2.

La présente divulgation permet ainsi avantageusement d'offrir un support d'accessoire 10 pour sonde ultrasonore assurant une fixation amovible d'un accessoire 20 à une sonde ultrasonore 2, cette fixation étant fiable et robuste tout en garantissant une bonne ergonomie d'utilisation et un nettoyage aisé, efficace et rapide.

En particulier, la configuration du support d'accessoire 10 et de l'accessoire 20 permet avantageusement une appairage mécanique par rotation de l'un par rapport à l'autre, ce qui facilite les manipulations par l'utilisateur (ergonomie améliorée) et permet de limiter le temps nécessaire à l'appairage et les risques de mauvais positionnement.

Un utilisateur peut aisément attacher et détacher l'accessoire 20 avec un bon compromis entre effort nécessaire et robustesse de maintien. Il est avantageusement possible de permuter entre divers accessoires 20 que l'on fixe à la sonde ultrasonore 2 au moyen du support d'accessoire 10.

La configuration particulière du support d'accessoire 10, et plus généralement du système accessoire SY1, permet avantageusement un nettoyage aisé, efficace et rapide de l'ensemble.

Par ailleurs, la nature de l'accessoire de sonde 20 peut varier selon le cas. Le support d'accessoire 10, et plus généralement le système accessoire SY1, peuvent en effet être adaptés pour permettre avantageusement de fixer divers accessoires de sonde 20 à une sonde ultrasonore 2.

A titre d'exemple, l'accessoire de sonde 20 comprend au moins l'un parmi :
- un capteur ;
- un distributeur de gel ; et
- une source lumineuse.

L'accessoire de sonde 20 peut ainsi être (ou comprendre) un capteur, et/ou un distributeur de gel et/ou une source lumineuse. D'autres types d'accessoire sont toutefois possibles.

Selon un exemple, l'accessoire de sonde 20 est un sujet (un humain ou un animal par exemple). Dans ce cas, le support d'accessoire 10 permet avantageusement de fixer de façon amovible la sonde ultrasonore 2 à un sujet, tel qu'un individu par exemple. Il est par exemple possible de fixer une telle sonde ultrasonore sur une partie anatomique d'un sujet afin de réaliser des tests, par exemple pour effectuer des scans échographiques.

Selon un exemple, l'accessoire 20 comprend un capteur, par exemple un capteur de position, de mouvement, de pression, optique, et/ou de température. L'accessoire 20 comprend par exemple un capteur de position (de type magnétique ou autre) configuré pour détecter la position de la sonde ultrasonore 2 dans l'espace lorsque l'accessoire 20 est fixé à la sonde ultrasonore 2 au moyen du support d'accessoire 10.

Selon un exemple, l'accessoire 20 comprend une source lumineuse (par exemple un dispositif d'éclairage) configurée pour éclairer une zone d'intérêt à proximité de la sonde ultrasonore 2 lorsque l'accessoire 20 est fixé à la sonde ultrasonore 2 au moyen du support d'accessoire 10.

Selon un exemple, l'accessoire 20 comprend un distributeur de gel configuré pour distribuer un gel, par exemple en vue d'être appliqué sur la sonde ultrasonore 2 et/ou sur une zone d'intérêt à proximité de la sonde ultrasonore 2.

Selon un exemple, l'accessoire de sonde 20 constitue un deuxième support, dit support de dispositif, configuré pour contenir ou supporter un dispositif accessoire, à savoir par exemple un capteur et/ou un distributeur de gel et/ou une source lumineuse tels que précédemment décrits.

Selon un exemple, l'accessoire 20 est un deuxième support comprenant un logement configuré pour contenir (ou recevoir) un capteur tel que précédemment décrit, par exemple un capteur de position (de type magnétique ou autre) et/ou un capteur de température.

Par ailleurs, l'accessoire de sonde 20 peut présenter diverses configurations de forme selon le cas. Selon un exemple, l'accessoire 20 forme un bouton rotatif. Ce bouton sert ainsi avantageusement d'élément préhensible qui peut être manipulé aisément (en particulier en rotation) lors de l'appairage mécanique AP2 de l'accessoire 20 avec le support d'accessoire 10.

Comme le comprend l'homme du métier, tous les modes de réalisation et variantes décrits ci-avant dont certains ont été simplifiés à dessein pour faciliter les explications, ne constituent que des exemples non limitatifs de mise en oeuvre de la présente divulgation. En particulier, l'homme du métier pourra envisager une quelconque adaptation ou combinaison des modes de réalisation et variantes décrits ci-avant, afin de répondre à un besoin particulier.

La présente invention ne se limite donc pas aux exemples de réalisation décrits ci-avant mais s'étend notamment à un procédé de pilotage qui inclurait des étapes secondaires sans pour cela sortir de la portée de la présente invention. Il en serait de même d'un dispositif de pilotage, ou plus généralement d'un système de pilotage, pour la mise en oeuvre d'un tel procédé.

## Revendications

1. Support d'accessoire (10) pour sonde ultrasonore comprenant :
- une première interface de fixation (12) configurée pour s'appairer mécaniquement à une sonde ultrasonore (2) ; et
- une deuxième interface de fixation (14), de forme complémentaire à une troisième interface de fixation (24) dont est pourvu un accessoire de sonde, configurée pour s'appairer mécaniquement (AP2) de façon amovible avec ladite troisième interface de fixation (24) par rotation (OP2) de l'accessoire de sonde relativement au support d'accessoire.

2. Support d'accessoire selon la revendication 1, dans lequel l'une parmi les deuxième et troisième interfaces de fixation (14, 24) constitue un élément mâle et l'autre constitue un élément femelle, lesdits éléments mâle et femelle étant configurés pour s'appairer mécaniquement (AP2) en rotation par insertion de l'élément mâle dans l'élément femelle.

3. Support d'accessoire selon la revendication 2, dans lequel la deuxième interface de fixation (14) du support d'accessoire (10) constitue l'élément femelle.

4. Support d'accessoire selon la revendication 2 ou 3, dans lequel la deuxième interface de fixation est configurée pour s'appairer mécaniquement de façon amovible avec ladite troisième interface de fixation par un appairage mécanique (AP2) comprenant successivement une insertion (OP1) de l'élément mâle dans l'élément femelle selon une direction d'insertion (6) et une rotation (OP2) de l'accessoire de sonde relativement au support d'accessoire selon un axe de rotation correspondant à la direction d'insertion (6).

5. Support d'accessoire selon l'une quelconque des revendications 2 à 4, dans lequel l'élément femelle comprend :
- au moins une encoche d'insertion (15) configurée pour que s'y insère un membre de verrouillage (26) respectif dont est pourvu l'élément mâle ; et
- une glissière de positionnement (16) débouchant respectivement sur chaque encoche d'insertion (15) pour permettre un déplacement, par rotation (OP2) de l'accessoire de sonde (20) relativement au support d'accessoire (10), du membre de verrouillage respectif dans la glissière de positionnement jusqu'à une position de verrouillage (P2) causant ainsi l'appairage mécanique (AP2) du support d'accessoire avec l'accessoire de sonde.

6. Support d'accessoire selon la revendication 5, dans lequel l'une parmi les deuxième et troisième interfaces de fixation (14, 24) comprend au moins une ailette déformable (17) configurée pour se déformer en réponse au déplacement du membre de verrouillage (26) respectif dans la glissière de verrouillage jusqu'à la position de verrouillage (P2) de sorte à causer une force de friction s'opposant audit déplacement.

7. Support d'accessoire selon la revendication 6, dans lequel ladite au moins une ailette déformable (17) est comprise dans la deuxième interface de fixation (14) du support d'accessoire (10).

8. Support d'accessoire selon la revendication 7, dans lequel la deuxième interface de fixation (14) comprend deux ailettes déformables (17).

9. Support d'accessoire selon l'une quelconque des revendications 6 à 8, dans lequel l'une parmi les deuxième et troisième interfaces de fixation (14, 24) comprend au moins une première cavité (27), ladite au moins une ailette déformable (17) et ladite au moins une première cavité (27) étant configurées pour que chaque ailette déformable s'engage dans une première cavité lorsque ledit au moins un membre de verrouillage (26) atteint la position de verrouillage (P2).

10. Support d'accessoire selon l'une quelconque des revendications 5 à 9, dans lequel la deuxième interface de fixation (14) comprend au moins deux encoches d'insertion (15) de taille différente configurées pour que s'y engagent des membres de verrouillage (26) respectifs de la troisième interface de fixation (24).

11. Support d'accessoire selon l'une quelconque des revendications 5 à 10, dans lequel l'une parmi les deuxième et troisième interfaces de fixation (14, 24) comprend une deuxième cavité (28) et l'autre comprend une protubérance (18) configurée pour s'engager dans la deuxième cavité lorsque ledit au moins un membre de verrouillage (26) atteint la position de verrouillage (P2).

12. Support d'accessoire selon l'une quelconque des revendications précédentes, dans lequel les deuxième et troisième interfaces de fixation (14, 24) sont configurées pour être appairées mécaniquement de façon amovible par une rotation de 1/4 de tour du support d'accessoire relativement à l'accessoire de sonde.

13. Système accessoire (SY1) pour sonde ultrasonore comprenant :
- un support d'accessoire (10) tel que défini dans l'une quelconque des revendications précédentes ; et
- un accessoire de sonde (20) comprenant une troisième interface de fixation (24) de forme complémentaire à la deuxième interface de fixation (14) du support d'accessoire, dans lequel les deuxième et troisième interfaces de fixation sont configurées pour s'appairer mécaniquement (AP2) de façon amovible par rotation (OP2) de l'accessoire de sonde relativement au support d'accessoire.

14. Système selon la revendication 13,
dans lequel l'une parmi les deuxième et troisième interfaces de fixation (14, 24) constitue un élément mâle et l'autre constitue un élément femelle, lesdits éléments mâle et femelle étant configurés pour s'appairer mécaniquement (AP2) en rotation par insertion de l'élément mâle dans l'élément femelle,
dans lequel les deuxième et troisième interfaces de fixation sont configurées pour s'appairer mécaniquement de façon amovible par un appairage mécanique (AP2) comprenant successivement une insertion (OP1) de l'élément mâle dans l'élément femelle selon une direction d'insertion (6) et une rotation (OP2) de l'accessoire de sonde relativement au support d'accessoire selon un axe de rotation correspondant à la direction d'insertion (6).

15. Système selon la revendication 13 ou 14, dans lequel l'accessoire de sonde (20) comprend au moins l'un parmi :
- un capteur ;
- un distributeur de gel ; et
- une source lumineuse.

16. Système selon la revendication 15, dans lequel l'accessoire de sonde (20) comprend un capteur de position.

17. Système selon l'une quelconque des revendication 13 à 16, dans lequel l'accessoire de sonde (20) forme un bouton rotatif.
